# EUROPEAN PATENT APPLICATION

(11) **EP 4 578 413 A1**
(43) Date of publication of application: **02.07.2025**
(21) Application number: 24216338.4
(22) Date of filing: 29.11.2024
(51) Int. Cl.: A61B 18/12, A61B 34/20

(54) **MEDICAL PROBE FOR NAVIGATING SMALL DIAMETER BLOOD VESSELS**

(30) Priority: 30.11.2023 US 202318525679
(71) Applicant: Biosense Webster (Israel) Ltd., 2066717 Yokneam (IL)
(72) Inventor: SUAREZ, Paul, Irvine, CA 92618 (US); HIGHSMITH, Debby, Irvine, CA 92618 (US); AMEFIA, Kokou Anani Mawuena, Irvine, CA 92618 (US); BAR-TAL, Meir, 2066717 Yokneam (IL); BERGER, Abraham, 2066717 Yokneam (IL)
(74) Representative: Carpmaels & Ransford LLP

(57) **Abstract**

The disclosed technology includes medical probe that includes an elongated shaft, a guidewire, coils, and electrodes. The elongated shaft extends along a longitudinal axis and is dimensioned to be inserted into a vein of Marshall. The guidewire extends through a lumen in the elongated shaft. The coils are connected to the distal dip of the elongated shaft and each coil is configured to generate a current when subjected to a magnetic field, the current being indicative of a position of the respective coil. The electrodes are connected to the distal tip. Each electrode is designed to (i) sense anatomical signals in the vein of Marshall and provide electrical signals which are indicative of the anatomical signals or (ii) convey ablation energy to a target tissue region proximal the vein of Marshall.

## Description

### FIELD

The present technology relates generally to medical devices, and in particular medical probes with electrodes, and further relates to, but not exclusively, medical probes suitable for use location tracking, anatomical sensing, and/or inducing irreversible electroporation (IRE) of cardiac tissues.

### BACKGROUND

The ligament of Marshall (LOM), which is located on the epicardium between the left atrial appendage and the left pulmonary veins, is often a source of paroxysmal atrial fibrillation (AF). Cardiac arrhythmias, such as AF, occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference.

Many regions of the heart are accessed endocardially relatively easily, and so are amenable to ablation by conventional catheters, such as a focal catheter or a balloon catheter. However, there are certain regions of the heart, such as the LOM, that are difficult to access endocardially, typically because a possible endocardial path is tortuous, comprising one or more relatively acute angular bends that a conventional catheter is unable to traverse. While these regions may in some cases be accessed epicardially, endocardial access is preferred.

### SUMMARY

There is provided, in accordance with the disclosed technology, a medical probe that includes an elongated shaft, a guidewire, a plurality of coils, and a plurality of electrodes. The elongated shaft extends along a longitudinal axis and is dimensioned to be inserted into a vein of Marshall. The elongated shaft comprises a distal tip and defines a lumen. The guidewire extends through the lumen. The coils are connected to the distal dip along the longitudinal axis, with each coil being configured to generate a current when subjected to a magnetic field. The current is indicative of a position of the respective coil. The electrodes are connected to the distal tip along the longitudinal axis, and each electrode is configured to (i) sense anatomical signals in the vein of Marshall and provide electrical signals which are indicative of the anatomical signals or (ii) convey ablation energy to a target tissue region proximal the vein of Marshall.

There is further provided, in accordance with the disclosed technology, a method for navigating a medical probe to a vein of Marshall. The method includes the step of: inserting a guidewire through a coronary sinus into the vein of Marshall. The method includes the step of: sliding an elongated shaft that extends along a longitudinal axis over the guidewire, through the coronary sinus, and into the vein of Marshall, with the elongated shaft comprising a distal tip, and with a plurality of coils and a plurality of electrodes being connected to the distal tip. The method includes the step of: generating a respective current in each coil of the plurality of coils by subjecting each coil to a magnetic field, the current being indicative of a position of the respective coil. The method includes the step of: positioning the distal tip within the vein of Marshall. The method includes the step of: sensing anatomical signals in the vein of Marshall with at least one electrode of the plurality of electrodes. The method includes the step of: providing an electrical signal from the at least one electrode which is indicative of the anatomical signals.

There is further provided, in accordance with the disclosed technology, a system includes a medical probe and a processor. The medical probe includes an elongated shaft, a guidewire, a plurality of coils, and a plurality of electrodes. The elongated shaft extends along a longitudinal axis and is dimensioned to be inserted into a vein of Marshall. The elongated shaft includes a distal tip and defines a lumen therethrough. The guidewire extends through the lumen. The plurality of coils are connected to the distal dip along the longitudinal axis, each coil being configured to generate a current when subjected to a magnetic field, the current being indicative of a position of the respective coil. The electrodes are connected to the distal tip along the longitudinal axis, with each electrode being configured to (i) sense anatomical signals in the vein of Marshall and provide electrical signals which are indicative of the anatomical signals or (ii) convey ablation energy to a target tissue region proximal the vein of Marshall. The processor is configured to estimate a position of the distal tip based on the generated currents and (i) estimate at least one characteristic of the anatomical signals based on the electrical signals or (ii) provide a signal to convey the ablation energy to the electrodes.

There is further provided, in accordance with the disclosed technology, a system including an elongated probe and a guidewire catheter. The elongated probe extends along a longitudinal axis and is dimensioned to be inserted into a coronary sinus. The elongated catheter shaft includes an opening. The guidewire catheter extends through the opening along the longitudinal axis and is dimension to be inserted into a vein of Marshall. A first plurality of electrodes is connected to the elongated probe along the longitudinal axis. A second plurality of electrodes is connected to the guidewire catheter along the longitudinal axis. Each electrode of the first plurality of electrodes and the second plurality of electrodes is configured to (i) generate a current that is indicative of a position of the respective electrode and (ii) sense anatomical signals of a tissue and provide electrical signals which are indicative of the anatomical signals.

There is further provided, in accordance with the disclosed technology, a method for navigating a medical probe to a vein of Marshall. The method includes the step of: inserting an elongated probe that extends along a longitudinal axis into a coronary sinus, with the elongated probe defining an opening and comprising a first plurality of electrodes. The method includes the step of: sliding a guidewire catheter through the elongated probe and out of the opening of the elongated probe into the vein of Marshall, with the guidewire catheter comprising a second plurality of electrodes. The method includes the step of: generating a respective current in each electrode of the first plurality of electrodes and the second plurality of electrodes, the respective current being indicative of a position of each respective electrode. The method includes the step of: sensing anatomical signals in the vein of Marshall with at least one electrode of the second plurality of electrodes. The method includes the step of: providing an electrical signal from the at least one electrode of the second plurality of electrodes which is indicative of the anatomical signals.

### BRIEF DESCRIPTION OF THE DRAWINGS

FIG. 1 is a schematic pictorial illustration of a medical system including a medical probe with a distal end that includes electrodes, in accordance with the disclosed technology;
FIG. 2 is a schematic pictorial illustration showing a perspective view of a medical probe with electrodes, with portions of an elongated probe body cut away, in accordance with the disclosed technology;
FIG. 3 is a schematic pictorial illustration showing a cross-sectional side view of the probe body, in accordance with the disclosed technology;
FIG. 4 is a schematic pictorial illustration showing the probe body inserted into the vein of Marshall, in accordance with the disclosed technology;
FIG. 5 is a schematic pictorial representation of a side elevation view of another medical probe, in accordance with the disclosed technology;
FIG. 6 is a schematic pictorial representation of Detail A in FIG. 5, in accordance with the disclosed technology;
FIG. 7 is a schematic pictorial representation of a cross sectional view of the distal tip the medical probe of FIG. 5, shown with a portion thereof cut away, in accordance with the disclosed technology;
FIG. 8 is a schematic pictorial representation of an outer portion of the distal tip of the medical probe of FIG. 5;
FIG. 9 is a schematic pictorial representation of an inner portion of the distal tip of the medical probe of FIG. 5;
FIG. 10 is a schematic pictorial illustration showing the probe body of FIG. 5 proximal the vein of Marshall and a guidewire inserted into the vein of Marshall, in accordance with the disclosed technology;
FIG. 11A is a schematic pictorial illustration showing the probe body of FIG. 5 inserted into the vein of Marshall, in accordance with the disclosed technology;
FIG. 11B is a schematic pictorial illustration, similar to FIG. 11A, showing the probe body of FIG. 5 inserted further into the vein of Marshall, in accordance with the disclosed technology;
FIG. 12 is a schematic pictorial representation of a side view of another medical probe, in accordance with the disclosed technology;
FIG. 13 is a schematic pictorial illustration showing the heart, with a guidewire catheter of the medical probe of FIG. 12 inserted into the vein of Marshall, in accordance with the disclosed technology;
FIG. 14A is a schematic pictorial representation of a side view of a modified tip of the guidewire catheter shown in FIGs. 12 and 13, in accordance with the disclosed technology; and
FIG. 14B is a schematic pictorial representation of Detail B shown in FIG. 14A, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the present disclosure. The detailed description illustrates by way of example, not by way of limitation, the principles of the disclosed technology. This description will clearly enable one skilled in the art to make and use the disclosed technology, and describes several embodiments, adaptations, variations, alternatives and uses of the disclosed technology, including what is presently believed to be the best mode of carrying out the disclosed technology.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular", "tube" and "shaft" are to be construed broadly and are not limited to a structure that is a right cylinder or strictly circumferential in cross-section or of a uniform cross-section throughout its length. For example, the tubular/shaft structures are generally illustrated as a substantially right cylindrical structure. However, the tubular/shaft structures may have a tapered or curved outer surface without departing from the scope of the present disclosure.

The present disclosure is related to systems, method or uses and devices for location tracking and mapping within the coronary sinus and/or vein of Marshall (VOM) and IRE ablation of cardiac tissue to treat cardiac arrhythmias. Ablative energies are typically provided to cardiac tissue (e.g., the LOM) by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized without the need for using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for irreversible electroporation (IRE), RF ablation, and/or cryoablation. IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The technology of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

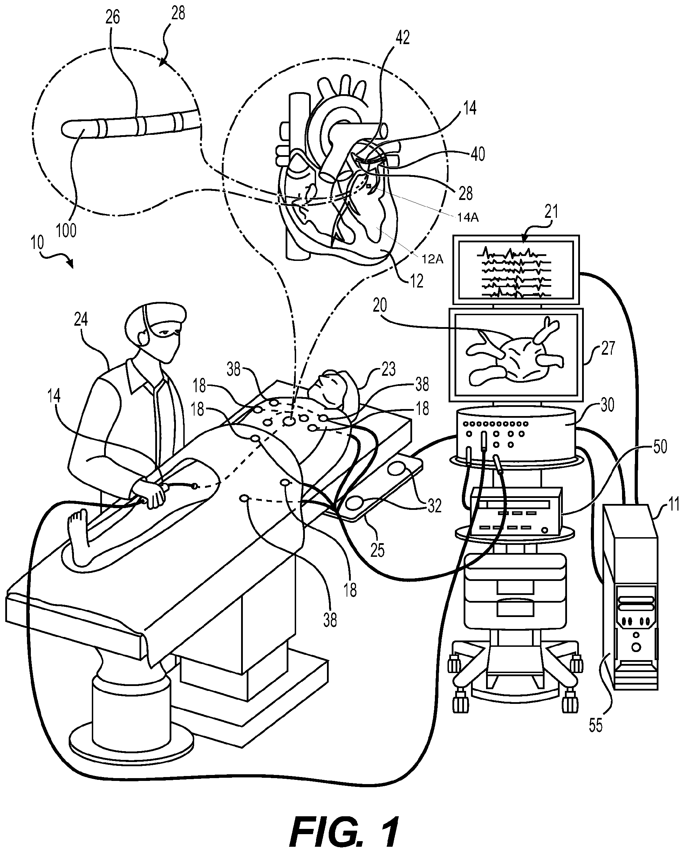

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, probe 14 is used to sense or ablate elements of the heart that are difficult to access, and by way of example, sensing anatomical signals and/or ablation of a portion of a LOM of heart 12 is described herein. However, it will be understood that medical probe 22 can be used, *mutatis mutandis,* for other therapeutic and/or diagnostic purposes in the heart or in other body organs. The plurality of catheters can include catheters dedicated for sensing Intracardiac Electrogram (IEGM) signals, catheters dedicated for ablating and/or catheters dedicated for both sensing and ablating. An example medical probe, e.g., a catheter 14, that is configured for sensing IEGM is illustrated herein. Physician 24 brings a distal tip of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over a probe body and configured to sense the IEGM signals. Catheter 14 can additionally include one or more position sensors embedded in or near the distal tip 28 for tracking position and orientation of distal tip 28. Optionally and preferably, position sensor is a magnetic based position sensor (discussed in greater detail below).

Magnetic based position sensor may be operated together with a location pad 25 including a plurality of magnetic coils 32 configured to generate magnetic fields in a predefined working volume. Real time position of the distal tip 28 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including unipolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

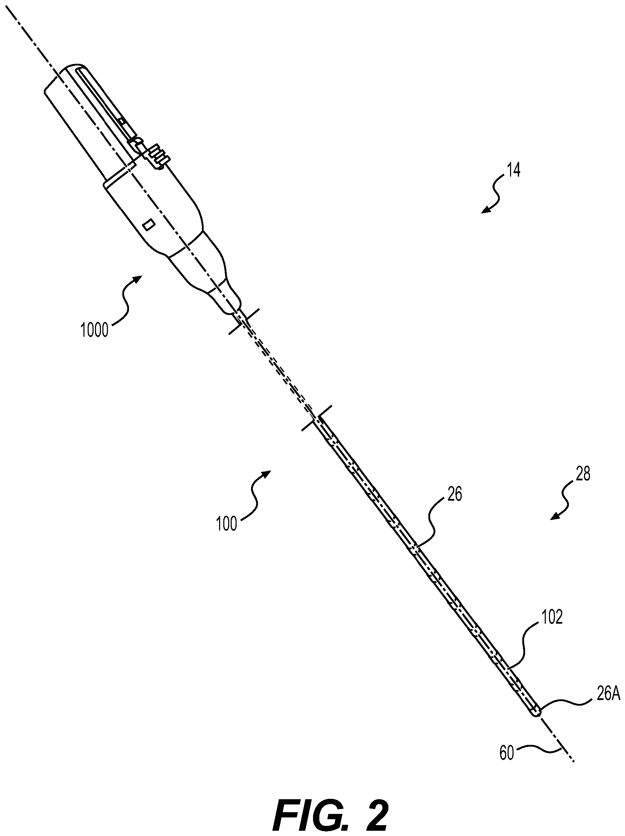

FIG. 2 is a schematic pictorial illustration showing a perspective view of a medical device 14 with electrodes 26. The medical device 14 includes a handle 1000 and a probe body 100 that extends from the handle 1000 along a longitudinal axis 60 and includes a distal tip 28. The probe body 100 in the presently described example includes an elongated flexible shaft 102. The shaft 102 can be formed from a flexible, biocompatible electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response.

In examples described herein, the electrodes 26 can be used to determine the location of the probe body 100 and/or to measure a physiological/anatomical property such as local surface electrical potentials at respective locations on tissue in heart 12. In addition to using electrodes 26 to determine the location and/or measure anatomical signals, electrodes 26 can also be configured to deliver ablation energy (IRE and/or RF) to tissue in heart 12. In some examples, the set of electrodes 26 can include a dome electrode 26A at a distal end of the probe body 100. As the lead electrode 26 at a distal end of the probe body 100, the dome electrode 26A can aid the physician 24 in navigating the catheter 14. The collected signals can be used to help guide the catheter 14, in general and particularly in cases where imaging is otherwise absent.

Examples of materials ideally suited for forming electrodes 26 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

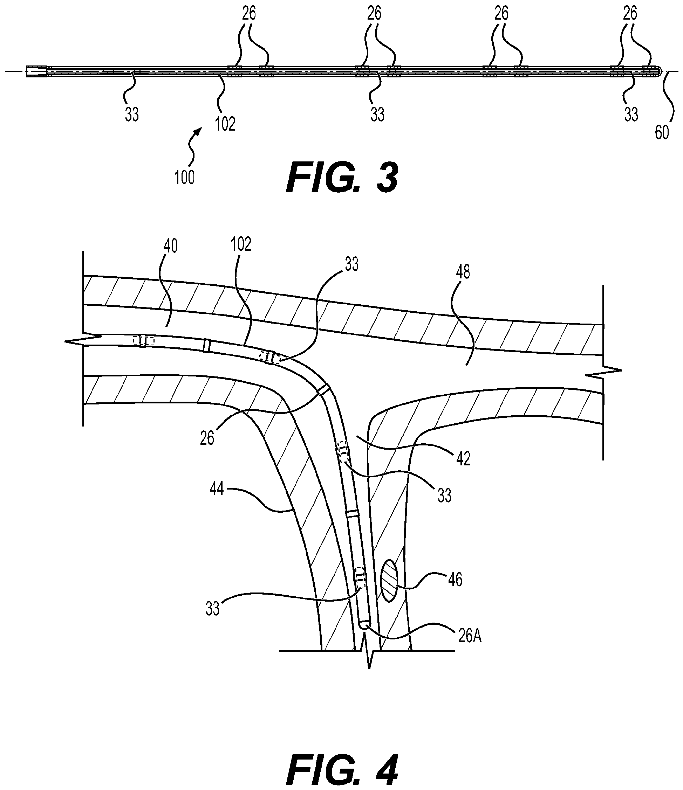

FIG. 3 is a schematic pictorial illustration showing a cross-sectional side view of the probe body 100. Embedded within the probe body 100 are a plurality of coils 33 disposed at predetermined locations along the longitudinal axis 60. Each coil 33 is configured to generate a current when subjected to a magnetic field. In some examples, each coil 33 can comprise a single axis sensor (SAS). The coils 33 can comprise a conductive material wound cylindrically in a coil, or a flat spiral coil formed into a planar flexible circuit. The coils 33 can comprise electrical leads for conduction of current induced on each coil 33 to the patient interface unit 30. As will be appreciated, by attaching a plurality of coils 33 to the distal tip 28 of the probe body 100, it is possible to detect a position thereof. In this way, a physician 24 can more accurately determine the position of the distal end of the probe body 100 before using it to sense anatomical signals and/or apply ablative energy to tissue in, e.g., the VOM 42.

FIG. 4 is a schematic pictorial illustration showing the coronary sinus 40, VOM 42, LOM 44, and great cardiac vein 48, with the probe body 100 inserted into the VOM 42. The following description assumes, by way of example, that a target region 46 of LOM 154 is to be mapped/sensed and/or ablated. It is noted that, for illustrative purposes, the coils 33 are illustrated in phantom lines in this figure.

In an initial step, physician 24 inserts the probe body 100 into the subject, and then navigates the probe body 100 into heart 12. Within the heart 12, the probe body is navigated via coronary sinus 40 into LOM 44 until a distal end of the probe body 100 is within proximity to target region 46. The distal end of the probe body 100 can be steered by, for example, one or more pull wires integrated therewith. The physician 24 is typically assisted in the navigation by processor 55 using signals from sensors 33, or from electrodes 26, to display an icon representing the location of the distal tip 28 of the probe body 100 on map 20. Additionally, physician 24 can use fluoroscopy to aid in the navigation.

In a mapping and/or sensing step, the location of electrodes 26 relative to target region 46 can be verified by a current tracking module of the PIU 30 using impedances and/or currents between the electrodes 26 and patches 38. Additionally, the location of the electrodes can be verified fluoroscopically. Once it has been determined that the electrodes 26 are positioned properly relative to the target region 46, medical probe body 100 is maneuvered to bring one or more of the electrodes 26 into contact with the intracardiac tissue, e.g., the inner heart surface. The electrodes 26 generate potential gradient signals in response to the sensed electrical potentials, also referred to herein as electrical signals. In some examples, the sensed electrical signals are indicative of a direction and propagation speed of wavefronts caused by anatomical signals, such as electrocardiogram (ECG) signals in the heart 12.

In an ablation step, the physician 24 operates processor 55 and ablation energy generator 50 to supply electrical current to electrodes 26. If there are two or more electrodes 26, then the supplied current can be bipolar, i.e., the current can be passed between the electrodes 26 so as to convey ablation energy to the tissue. Alternatively, the supplied ablation energy can be unipolar, i.e., an electrical current may be applied between one of electrodes 26 and a return electrode connected to generator 50. The return electrode can be disposed outside the body of patient 23. For example, the return electrode may comprise a patch (e.g., electrode patch 38 or the like) coupled to the patient's body.

In some examples, RF sinusoidal-like (alternating) electrical current is supplied to the electrodes 26, such that RF ablation of the tissue is performed. Alternatively, pulsed current (direct current) may be supplied so as to perform irreversible electroporation (IRE) or pulsed field ablation (PFA). When pulsed current is supplied to perform PFA, unipolar ablation energy can be supplied by direct electrical current running between the electrodes 26 on the elongated shaft 202 and the electrode patch(es) 38 or a backpatch. Moreover, bipolar ablation energy can be supplied by direct electrical current running between the electrodes 26 on the elongated shaft 202 themselves and/or another catheter 14A (e.g., a focal catheter or a multielectrode catheter) within the left atrium 12A (see FIG. 1).

Once the physician 24 completes the mapping and/or ablation, the physician can withdraw the medical probe body 100 from patient 23. The previously described method describes the mapping and/or ablation of one target region. Of course, implementations of the presently described technology are not limited to sensing and/or ablation of a single region, but rather may be used to map/sense and/or ablate two or more separate regions during a single ablation procedure. For example, if there is a second target region, closer to the coronary sinus 40 than target region 46, the electrodes 26 can be moved into proximity with the second region, and the electrodes 26 may be pushed to contact the second region in preparation for sensing anatomical signals and/or ablation of that region.

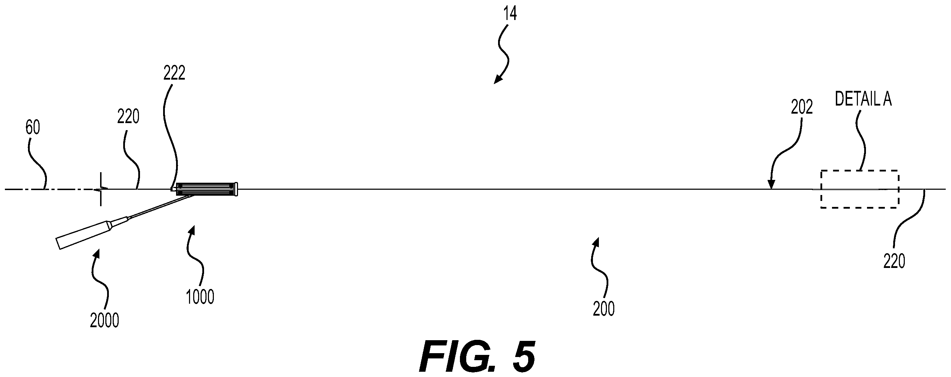

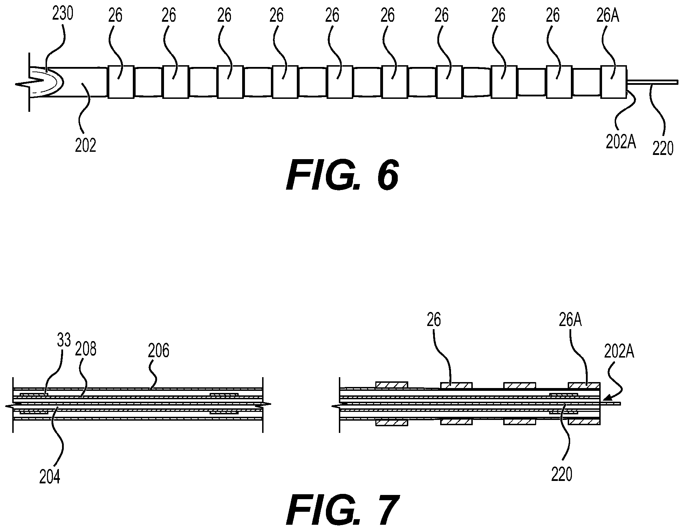

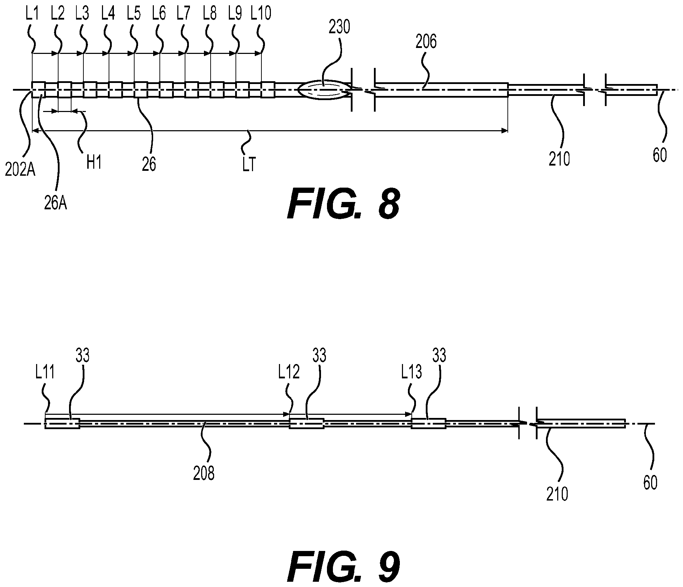

FIG. 5 is a schematic pictorial representation of a side view of another medical probe 14 designed in accordance with the presently disclosed technology. FIG. 6 is a schematic pictorial representation of a detail view of Detail A of FIG. 5. FIG. 7 is a schematic pictorial representation of a cross sectional view of the distal tip the medical probe 14 of FIG. 5, shown with a portion thereof cut away. FIG. 8 is a schematic pictorial representation of an outer portion of the distal tip of the medical probe 14 of FIG. 5. FIG. 9 is a schematic pictorial representation of an inner portion of the distal tip of the medical probe 14 of FIG. 5.

Making reference to FIGs. 5-9, the medical device 14 includes a probe body 200 that extends parallel to or coaxial with a longitudinal axis 60 and includes a distal tip 28. The probe body 200 is connected to a handle 1000. The handle 1000 can also connect with an energy connector housing 2000, through which energy, such as PFA, can be routed for ablative purposes. The probe body 200 in the presently described example includes an elongated flexible shaft 202, defines a lumen 204, and is dimensioned to be inserted into the VOM 46. By way of example, an outer diameter of the shaft 202 can be approximately 3-4 Fr, e.g., .04-.05 inches.

As seen particularly in FIG. 7, the elongated shaft 202 includes a tubular outer wall 206 and a tubular lumen wall 208 that are coaxial with one another. The lumen wall 208 is nested within the outer wall 206 and at least partially defines the lumen 204. The outer wall 206 and lumen wall 208 are attached to a connecting shaft 210 that runs to the handle 1000. The outer wall 206, lumen wall 208, and connecting shaft 210 can each be formed from a flexible, biocompatible electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response.

As shown in FIGs. 5-7, a guidewire 220 extends through a Luer hub 222 in the handle 1000, through the lumen 204, and out a distal end 202A of the elongated shaft 202A. To fit through the lumen 204, the guidewire 220 can have a very small diameter, such as one within the range of .01 and .02 inches. It will be understood that the intra-cardiac navigation to a difficult to access site such as LOM44 is complicated, since guidewire 220 has to bend around one or more acute angles. For example, to reach the LOM 44, the guidewire 220 may need to pass through the inferior vena cava, right atrium, and coronary sinus 40. The complicated navigation is facilitated by guidewire 220 being configured to be extremely thin yet constructed to be flexible without kinking.

Similar to the previously described example, the elongated shaft 202, at its distal tip, includes electrodes 26 connected thereto. Specifically, the electrodes 26 can be designed as ring electrodes 26 that extend around the outer wall 208 and coaxial to and spaced along the longitudinal axis 60. The ring electrodes 26 can have a similar outer diameter as that of the elongated shaft 202 (e.g., between .04 and .05 inches), such that they are either substantially flush or bulge slightly from the elongated shaft 202. A distalmost electrode 26A can be designed either as a ring electrode or a dome electrode. As the lead electrode 26 at a distal end of the elongated shaft, the distalmost electrode 26A can aid the physician 24 in navigating the catheter 14. The collected signals can be used to help guide the catheter 14, in general and particularly in cases where imaging is otherwise absent. As discussed above, in examples described herein, the electrodes 26 can be used to determine the location of the probe body 200 and/or to measure a physiological/anatomical property such as local surface electrical potentials at respective locations on tissue in heart 12. In addition to using electrodes 26 to determine the location and/or measure anatomical signals, electrodes 26 can also be configured to deliver ablation energy (IRE and/or RF) to tissue in heart 12.

In some examples, approximately eight to ten electrodes 26 can be provided on the elongated shaft 202 that have a predetermined spacing relative to one another along the longitudinal axis 60. With specific reference to FIG. 8, the presently described example includes ten electrodes 26, each electrode 26 having a height H1 and a predetermined spacing length L1-L10 relative to the distal end 202A of the elongated shaft 202. Solely by way of example, and not to limit the scope of the present disclosure, the electrodes 26 may have a height H1 of approximately 1 millimeter and a spacing of 2 millimeters from one another (e.g., L1, which is the location of the distal end of the distalmost electrode 26, is approximately flush with, i.e., 0 millimeters from, the distal end 202A; L2, which is the distal end of the second distalmost electrode 26, is approximately 2 millimeters therefrom; L3, which is the distal end of the third distalmost electrode 26, is approximately 4 millimeters therefrom, etc.). Moreover, the outer wall 206 has a total length of LT. In some examples, LT is approximately 130 millimeters.

Further to the above, and as seen in FIGs. 7 and 9, embedded within the probe body 200 are a plurality (such as three) of coils 33 disposed at predetermined locations along the longitudinal axis 60. Each coil 33 is configured to generate a current when subjected to a magnetic field. In some examples, each coil 33 can comprise a single axis sensor (SAS). The coils 33 can comprise a conductive material wound in a coil, or a coil formed into a flexible circuit. The coils 33 can comprise electrical leads for conduction of current induced on each coil 33 to the patient interface unit 30. As will be appreciated, by attaching a plurality of coils 33 to the distal tip 28 of the probe body 100, it is possible to detect a position thereof. In this way, a physician 24 can more accurately determine the position of the distal end of the probe body 100 before using it to sense anatomical signals and/or apply ablative energy to tissue in, e.g., the VOM 42.

With continued reference to FIGs. 7 and 9, each coil 33 is mounted on the lumen wall 208 such that each coil 33 is disposed between (i.e., sandwiched between) the outer wall 206 and the lumen wall 208. Like the electrodes 26, a distal end of each coil 33 has a predetermined position (L11, L12, and L13, respectively) relative to the distal end 202A of the elongated shaft 202. Specifically, and for example, the distal end L11 of the distalmost coil 33 is approximately flush with (i.e., approximately 0 millimeters from) the distal end 202A of the elongated shaft 202, the distal end L12 of the middle coil 33 is approximately 24 millimeters away from the distal end 202A, and the distal end L13 of the most proximal coil 33 is approximately 36 millimeters away from the distal end 202A. Accordingly, it will be appreciated that, with the aforementioned exemplary predetermined positions, the distalmost coil 33 overlaps with the distalmost electrode 26 along the longitudinal axis 60, while the middle and most proximal coils 33 are sufficiently spaced from the distal end 202A of the elongated shaft 202 such that they do not overlap with any of the electrodes 26 and are both positioned more proximally along the longitudinal axis 60 than all of the electrodes 26.

Of course, other coil 33 configurations may be appropriate. For example, two coils 33 (rather than three) may be sufficient for tracking the location of the distal tip 28. As another example, rather than positioning the middle and most proximal coils 33 such that they do not overlap with any of the electrodes 26 along the longitudinal axis 60, some or all of the coils 33 can be positioned such that they overlap with positions of the electrodes 26 (similar to as depicted in FIG. 4).

Additionally, in some examples, the elongated shaft 202 can also include an inflatable balloon 230. As shown in FIG. 8 (the balloon 230 is uninflated in this drawing), the balloon 230 is provided more proximal to the handle 1000 than some or all of the electrodes 26, that is inflatable via a balloon lumen extending through the elongated shaft 202, for purposes that are discussed in greater detail below.

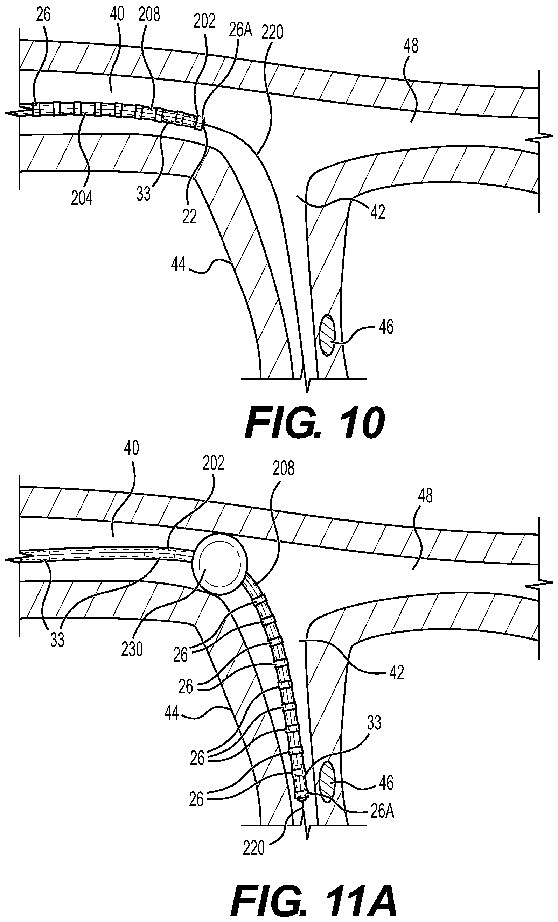

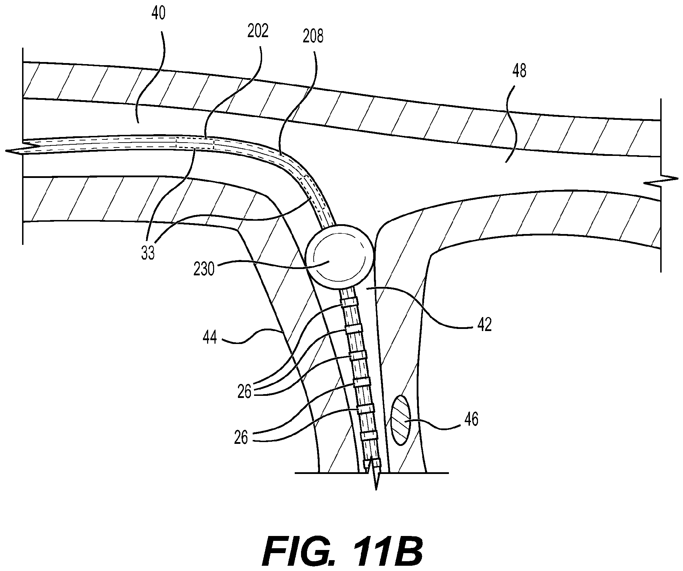

FIG. 10 is a schematic pictorial illustration showing the coronary sinus 40, VOM 42, LOM 44, and great cardiac vein 48, with the probe body 200 proximal the VOM 42 and the guidewire 220 inserted into the VOM 42. FIG. 11A is a schematic pictorial illustration showing the probe body 200 inserted into the VOM 42. FIG. 11B is a schematic pictorial illustration showing the probe body 200 inserted further into the VOM 42 than the depiction of FIG. 11A. The following description assumes, by way of example, that a target region 46 of LOM 154 is to be mapped/sensed and/or ablated. Of course, it will be appreciated that the target region 46 can be of various sizes and forms, and not necessarily as depicted in the figures. It is noted that, for illustrative purposes, the lumen 204 and coils 33 are illustrated in phantom lines in these figures.

In an initial step, physician 24 inserts the probe body 200 and guidewire 220 into the subject, and then navigates the guidewire 220 into heart 12. Within the heart 12, the probe body 200 and guidewire 220 are navigated via the coronary sinus 40 until a distal end of the probe body 200 is within proximity to an entrance of the VOM 42 (e.g., as shown in FIG. 10). The location of the entrance to the VOM 42 can be determined by the physician 24 using the signals received from the coils 33 and/or one or more of the electrodes 26. In that way, the physician 24 is assisted in the navigation by processor 55 using those signals from sensors 33, or from electrodes 26, to display an icon representing the location of the distal tip 28 of the probe body 200 on map 20. Additionally, physician 24 can use fluoroscopy to aid in the navigation.

In a guidewire 220 insertion step, and as shown in FIG. 10, once the entrance to the VOM 42 has been located, the guidewire 220 is extended distally out of the lumen 204 and routed into the VOM 42. The guidewire 220 reduces the complexity of entering the VOM 42, which, as mentioned above, is difficult to access.

In a probe body 200 insertion step, and as shown in FIG. 11A, once the guidewire 220 has been routed into the VOM 42, a distal tip 28 of the elongated shaft 202 is slid along the guidewire 220 into the VOM 42 until it reaches its target region 46. Additionally, or alternatively, the elongated shaft 202 can be further inserted into the VOM 42, as shown in FIG. 11B.

In a balloon inflation step, and as shown in FIGs. 11A and 11B, the balloon 230 can optionally be inflated to occlude the coronary sinus 40 and/or great cardiac vein 48 and/or VOM 42, depending on placement. As shown in FIG. 11A, the balloon 230 occludes the coronary sinus 40. Further inserting the elongated shaft 202 into the VOM 42, as shown in FIG. 11B, positions the balloon 230 such that it can be inflated to occlude the VOM 42. This aids in maintaining the probe body 200 in the desired position and also prevents the flow of fluid in the occluded vessel in scenarios where a foreign fluid (e.g., ethanol or saline) is injected into the vessel via the lumen 204, which is discussed in greater detail below. Because of its purpose (location retention and/or occlusion), no complex designs thereof are required (e.g., no electrodes are positioned on the balloon 230 since it is not directly being used for mapping and/or ablation).

In a mapping and/or sensing step, the location of electrodes 26 relative to target region 46 can be verified by a current tracking module of the PIU 30 using impedances and/or currents between the electrodes 26 and patches 38, as well as (or alternatively) using the coils 33. Additionally, the location of the electrodes can be verified fluoroscopically. Once it has been determined that the electrodes 26 are positioned properly relative to the target region 46 (e.g., by generating a respective current in each coil 33 by subjecting each coil to a magnetic field, the current being indicative of a position of the respective coil 33), medical probe body 200 is maneuvered to bring one or more of the electrodes 26 into contact with the intracardiac tissue, e.g., the inner heart surface. The electrodes 26 generate potential gradient signals in response to the sensed electrical potentials, also referred to herein as electrical signals. In some examples, the sensed electrical signals are indicative of at least one characteristic of the anatomical signals, such as a direction and propagation speed of wavefronts caused by anatomical signals, such as electrocardiogram (ECG) signals in the heart 12.

In an ablation step, the physician 24 operates processor 55 and ablation energy generator 50 to supply electrical current to electrodes 26. If there are two or more electrodes 26, then the supplied current can be bipolar, i.e., the current can be passed between the electrodes 26 so as to convey ablation energy to the tissue. Alternatively (e.g., if there is only one electrode 26), the supplied ablation energy can be unipolar, i.e., an electrical current may be applied between one of electrodes 26 and a return electrode connected to generator 50. The return electrode can be disposed outside the body of patient 23. For example, the return electrode may comprise a patch (e.g., electrode patch 38 or the like) coupled to the patient's body.

In some examples, RF sinusoidal-like (alternating) electrical current is supplied to the electrodes 26, such that RF ablation of the tissue is performed. Alternatively, pulsed current (direct current) may be supplied so as to perform irreversible electroporation (IRE) or pulsed field ablation (PFA). When pulsed current is supplied to perform PFA, unipolar ablation energy can be supplied by direct electrical current running between the electrodes 26 on the elongated shaft 202 and the electrode patch(es) 38 or a backpatch. Moreover, bipolar ablation energy can be supplied by direct electrical current running between the electrodes 26 on the elongated shaft 202 themselves and/or another catheter 14A (e.g., a focal catheter or a multielectrode catheter) within the left atrium 12A (see FIG. 1).

Additionally, or alternatively, in some examples, the lumen 204 can be used to route ethanol therethrough that performs chemical ablation of the LOM 44. In such examples, the balloon 230 that is proximal the electrodes 26 is inflated (as discussed above) to occlude the VOM 42, as shown in FIG. 11B. FIGs. 11A and 11B depict exemplary positions in which the balloon 230 can be maneuvered to occlude different vessels of the heart 12. Moreover, saline can also be routed through the lumen 204 for use as a virtual electrode. The inflated balloon 230 prevents the injected fluid from draining before ablation can be performed.

Once the physician 24 completes the mapping/sensing and/or ablation, the physician can withdraw the medical probe body 200 and the guidewire from patient 23. The foregoing method describes the mapping/sensing and/or ablation of one target region. Of course, implementations of the presently described technology are not limited to sensing and/or ablation of a single region, but rather may be used to map/sense and/or ablate two or more separate regions (or an entire vessel) during a single ablation procedure. For example, if there is a second target region, closer to the coronary sinus 40 than target region 46, the electrodes 26 can be moved into proximity with the second region, and the electrodes 26 may be pushed to contact the second region in preparation for sensing anatomical signals and/or ablation of that region.

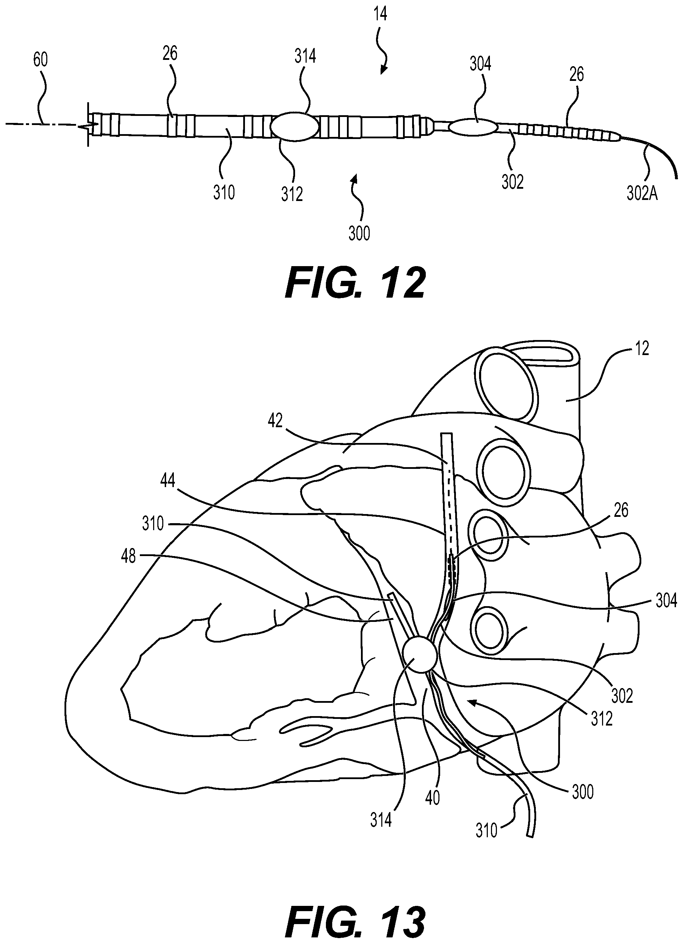

FIG. 12 is a schematic pictorial representation of a side view of another medical probe 14 designed in accordance with the presently disclosed technology. FIG. 13 is a schematic pictorial illustration showing the heart 12, with an elongated probe 310 (embodied as a shaft) proximal the VOM 42 and a guidewire catheter 302 inserted into the VOM 42.

Making reference to FIG. 12, the medical device 14 includes a probe body 300 that extends parallel to or coaxial with a longitudinal axis 60 and includes a distal tip 28. The probe body 200 is connected to a handle 1000 (not specifically depicted in this example, but it is understood that it may take the form of previously described handles 1000). The handle 1000 can also connect with an energy connector housing, through which energy, such as PFA, can be routed for ablative purposes.

The probe body 300 in the presently described example includes an elongated coronary sinus catheter embodied as an elongated probe 310 extending along (e.g., coaxial with) a longitudinal axis 60. The elongated probe 310 defines a primary lumen, a distal end opening (right side of FIG. 12), a side opening 312, and is dimensioned to be inserted into the coronary sinus 40. By way of example, an outer diameter of the coronary sinus catheter 310 can be approximately 6-7 Fr, e.g., .08-.09 inches.

The elongated probe 310 includes a plurality of electrodes 26 (e.g., ring electrodes substantially flush with or bulging from the probe 310) connected to elongated probe 310 along the longitudinal axis 60. The elongated probe 310 can also include a probe balloon 314 that is inflatable via a balloon lumen extending through the elongated probe 310, and is discussed in greater detail below.

As shown in FIG. 12, extending through the primary lumen of the elongated probe 310 along the longitudinal axis 60 (e.g., coaxial with) is a guidewire catheter 302 that is dimensioned to be inserted into the vein of Marshall 42. In use, the guidewire catheter 302 can be extended through the distal end opening (FIG. 12) or through the side opening 312 (FIG. 13) of the elongated probe 310. Compared with the previously described guidewire 220, this guidewire catheter 302 has a larger outer diameter. For example, the guidewire catheter 302 in the present example has an outer diameter of approximately .03 to .04 inches (2-3 Fr). Through the guidewire catheter 302 is a guidewire lumen (embodied similarly to the lumen 204 previously described) through which a guidewire 302A (which has a diameter of approximately, e.g., .010-.014 inches) passes, which aids the guidewire catheter 302 with intra-cardiac navigation, and is discussed in greater detail below.

The guidewire catheter 302 includes a plurality of electrodes 26 (e.g., ring electrodes substantially flush with or bulging slightly from the catheter 302) connected thereto along the longitudinal axis 60. The guidewire catheter 302 can also include a distalmost electrode 26A as previously described. The guidewire catheter 302 can also include a guidewire balloon 304, provided more proximal to the handle 1000 than some or all of the electrodes 26, that is inflatable via a balloon lumen extending through the guidewire catheter 302, and is discussed in greater detail below.

The probe 310 and guidewire catheter 302 can each be formed from a flexible, biocompatible electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response.

FIG. 13 depicts an exemplary use of the probe body 300. In an initial step, physician 24 inserts the elongated probe 310 and guidewire catheter 302 (which is retracted into the coronary sinus catheter 310) into the subject, and then navigates them into heart 12. Within the heart 12, the coronary sinus catheter 310 and guidewire catheter 302 are navigated via the coronary sinus 40 until a distal end of the coronary sinus catheter 310 is within proximity to an entrance of the VOM 42. The location of the entrance to the VOM 42 can be determined by the physician 24 using the signals received from the electrodes 26 of the elongated probe 310 and/or the guidewire catheter 302. In that way, the physician 24 is assisted in the navigation by processor 55 using those signals from electrodes 26, to display an icon representing the location of the distal tip 28 of the probe body 300 on map 20. Additionally, physician 24 can use fluoroscopy to aid in the navigation.

In a balloon inflation step, and as shown in FIG. 13, the probe balloon 314 can optionally be inflated to occlude the coronary sinus 40 and/or great cardiac vein 48, depending on placement. This aids in maintaining the elongated probe 310 in the desired position and also prevents the flow of fluid in the occluded vessel in scenarios where a foreign fluid (e.g., ethanol or saline) is injected into the vessel via the guidewire catheter 302, which is discussed in greater detail below. Because of its purpose (location retention and/or occlusion), no complex designs thereof are required (e.g., no electrodes are positioned on the probe balloon 314 since it is not directly being used for mapping and/or ablation).

In a guidewire catheter 302 insertion step, and as shown in FIG. 13, once the entrance to the VOM 42 has been located, depending on the orientation and/or position of the elongated probe 310, the guidewire 302A is first extended distally out of the distal end opening or side opening 312 (in FIG. 13, the guidewire catheter 302 exits the side opening 312) and routed into the VOM 42, followed by the guidewire catheter 302 being slid over the guidewire 302A. In this way, the guidewire 302A aids with maneuvering the guidewire catheter 302 out one of the openings of the elongated probe 310 and into the VOM 42, which, as mentioned above, is difficult to access. Once the VOM 42 has been accessed, if needed, the guidewire balloon 304 can be inflated to occlude the VOM 42 and/or maintain the guidewire catheter 302 in position. Because of its purpose (location retention and/or occlusion), no complex designs thereof are required (e.g., no electrodes are positioned on the balloon 304 since it is not directly being used for mapping and/or ablation).

In a mapping and/or sensing step, the location of electrodes 26 on the guidewire catheter 302 relative to a target region can be verified by a current tracking module of the PIU 30 using impedances and/or currents between the electrodes 26 and patches 38. Additionally, the location of the electrodes can be verified fluoroscopically. Once it has been determined that the electrodes 26 are positioned properly relative to the target region, guidewire catheter 302 is maneuvered to bring one or more of the electrodes 26 into contact with the intracardiac tissue, e.g., the inner heart surface. The electrodes 26 generate potential gradient signals in response to the sensed electrical potentials, also referred to herein as electrical signals. In some examples, the sensed electrical signals are indicative of at least one characteristic of the anatomical signals, such as a direction and propagation speed of wavefronts caused by anatomical signals, such as electrocardiogram (ECG) signals in the heart 12.

In an ablation step, the physician 24 operates processor 55 and ablation energy generator 50 to supply electrical current to electrodes 26 of the guidewire catheter 302. If there are two or more electrodes 26, then the supplied current can be bipolar, i.e., the current can be passed between the electrodes 26 so as to convey ablation energy to the tissue. Alternatively, the supplied ablation energy can be unipolar, i.e., an electrical current may be applied between one of electrodes 26 and a return electrode connected to generator 50. The return electrode can be disposed outside the body of patient 23. For example, the return electrode may comprise a patch (e.g., electrode patch 38 or the like) coupled to the patient's body.

In some examples, RF sinusoidal-like (alternating) electrical current is supplied to the electrodes 26, such that RF ablation of the tissue is performed. Alternatively, pulsed current (direct current) may be supplied so as to perform irreversible electroporation (IRE) or pulsed field ablation (PFA). When pulsed current is supplied to perform PFA, unipolar ablation energy can be supplied by direct electrical current running between the electrodes 26 on the guidewire catheter 302 and the electrode patch(es) 38 or a backpatch. Moreover, bipolar ablation energy can be supplied by direct electrical current running between the electrodes 26 on the guidewire catheter 302 themselves and/or electrodes 26 on the elongated probe 26 and/or another catheter 14A (e.g., a focal catheter or a multielectrode catheter) within the left atrium 12A (see FIG. 1).

Additionally, or alternatively, in some examples, the guidewire catheter 302 the narrow diameter guidewire lumen can be used to route ethanol therethrough that performs chemical ablation of the LOM 44. In such examples, the guidewire balloon 304 is inflated to occlude the VOM 42. Moreover, saline can also be routed through the guidewire lumen for use as a virtual electrode. The inflated balloon 304 prevents the injected fluid from draining before ablation can be performed.

Once the physician 24 completes the mapping/sensing and/or ablation, the physician can withdraw the medical probe body 300 and the guidewire from patient 23. The foregoing method describes the mapping/sensing and/or ablation of one target region. Of course, implementations of the presently described technology are not limited to sensing and/or ablation of a single region, but rather may be used to map/sense and/or ablate two or more separate regions (or an entire vessel) during a single ablation procedure. For example, if there is a second target region, closer to the coronary sinus 40 than target region 46, the electrodes 26 can be moved into proximity with the second region, and the electrodes 26 may be pushed to contact the second region in preparation for sensing anatomical signals and/or ablation of that region.

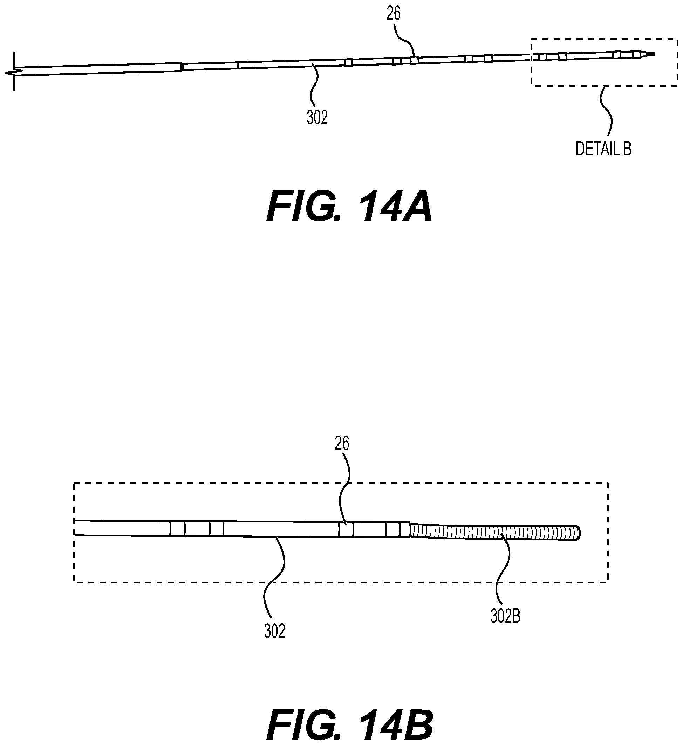

FIG. 14A is a schematic pictorial representation of a side view of a modified version of the guidewire catheter 302 described with respect to FIGs. 12 and 13. FIG. 14B is a schematic pictorial representation of a detail view of Detail B shown in FIG. 14A. The guidewire catheter 302 depicted in these figures is the same as previously described, with the exception that, rather than a guidewire lumen being defined therethrough, the guidewire 302A may be substituted with or further provided with an atraumatic material 302B at a distal end of the guidewire catheter 302, the atraumatic material 302B functioning as a guidewire tip.

The disclosed technology described herein can be further understood according to the following clauses:
Clause 1. A medical probe comprising: an elongated shaft that extends along a longitudinal axis and is dimensioned to be inserted into a vein of Marshall, the elongated shaft comprising a distal tip and defining a lumen; a guidewire that extends through the lumen; a plurality of coils connected to the distal dip along the longitudinal axis, each coil being configured to generate a current when subjected to a magnetic field, the current being indicative of a position of the respective coil; and a plurality of electrodes connected to the distal tip along the longitudinal axis, each electrode being configured to (i) sense anatomical signals in the vein of Marshall and provide electrical signals which are indicative of the anatomical signals or (ii) convey ablation energy to a target tissue region proximal the vein of Marshall.
Clause 2. The medical probe of clause 1, the elongated shaft being coaxial with the longitudinal axis.
Clause 3. The medical probe of any one of clauses 1-2, each electrode comprising a ring electrode comprising an electrode axis coaxial with the longitudinal axis.
Clause 4. The medical probe of any one of clauses 1-2, the plurality of electrodes further comprising a dome electrode connected to a distal end of the distal tip.
Clause 5. The medical probe of any one of clauses 1-2, the plurality of electrodes comprising eight to ten ring electrodes that are positioned, relative to a distal end of the distal tip, in approximately two-millimeter increments from one another along the longitudinal axis.
Clause 6. The medical probe of any one of clauses 1-5, a first coil of the plurality of coils being disposed within the distal tip and positioned so as to overlap with a distalmost electrode of the plurality of electrodes along the longitudinal axis.
Clause 7. The medical probe of clause 6, the plurality of coils further comprising a second coil and a third coil, the second coil and the third coil being positioned away from the distal end of the distal tip such that the second coil and the third coil are non-overlapping with each electrode of the plurality of electrodes.
Clause 8. The medical probe of any one of clauses 1-7, the elongated shaft comprising an outer wall and a lumen wall that defines the lumen.
Clause 9. The medical probe of clause 8, each coil being disposed between the outer wall and the lumen wall.
Clause 10. The medical probe of any one of clauses 1-9, the elongated shaft having an outer diameter between approximately .04 inches and .05 inches.
Clause 11. The medical probe of any one of clauses 1-10, each coil comprising a single axis sensor selected from a group consisting of a flat spiral coil, cylindrical coil or combination thereof.
Clause 12. The medical probe of any one of clauses 1-11, the guidewire having a diameter between approximately .01 and .02 inches.
Clause 13. The medical probe of any one of clauses 1-12, each electrode being configured to (i) sense the anatomical signals in the vein of Marshall and provide the electrical signals which are indicative of the anatomical signals and (ii) convey the ablation energy to the target tissue region proximal the vein of Marshall.
Clause 14. A method for navigating a medical probe to a vein of Marshall, the method comprising: inserting a guidewire through a coronary sinus into the vein of Marshall; sliding an elongated shaft that extends along a longitudinal axis over the guidewire, through the coronary sinus, and into the vein of Marshall, the elongated shaft comprising a distal tip, with a plurality of coils and a plurality of electrodes being connected to the distal tip; generating a respective current in each coil of the plurality of coils by subjecting each coil to a magnetic field, the current being indicative of a position of the respective coil; positioning the distal tip within the vein of Marshall; sensing anatomical signals in the vein of Marshall with at least one electrode of the plurality of electrodes; and providing an electrical signal from the at least one electrode which is indicative of the anatomical signals.
Clause 15. The method of clause 14, the anatomical signals comprising electrocardiogram signals.
Clause 16. The method of any one of clauses 14-15, further comprising: conveying ablation energy to a target tissue region proximal the vein of Marshall.
Clause 17. The method of clause 16, the ablation energy comprising unipolar ablation energy that is conveyed by routing direct electrical current between at least one electrode of the plurality of electrodes and an electrode patch.
Clause 18. The method of clause 16, the ablation energy comprising bipolar ablation energy that is conveyed by routing direct electrical current in a manner selected from the group consisting of: between at least one electrode of the plurality of electrodes and another electrode of the plurality of electrodes, between at least one electrode of the plurality of electrodes and a catheter positioned within an atrium, and combination thereof.
Clause 19. A system comprising: a medical probe comprising: an elongated shaft that extends along a longitudinal axis and is dimensioned to be inserted into a vein of Marshall, the elongated shaft comprising a distal tip and defining a lumen therethrough; a guidewire that extends through the lumen; a plurality of coils connected to the distal dip along the longitudinal axis, each coil being configured to generate a current when subjected to a magnetic field, the current being indicative of a position of the respective coil; and a plurality of electrodes connected to the distal tip along the longitudinal axis, each electrode being configured to (i) sense anatomical signals in the vein of Marshall and provide electrical signals which are indicative of the anatomical signals or (ii) convey ablation energy to a target tissue region proximal the vein of Marshall; and a processor configured to estimate a position of the distal tip based on the generated currents and (i) estimate at least one characteristic of the anatomical signals based on the electrical signals or (ii) provide a signal to convey the ablation energy to the electrodes.
Clause 20. The system of clause 19, the elongated shaft being coaxial with the longitudinal axis.
Clause 21. The system of any one of clauses 19-20, each electrode comprising a ring electrode comprising an electrode axis coaxial with the longitudinal axis.
Clause 22. The system of any one of clauses 19-21, further comprising a dome electrode connected to a distal end of the distal tip.
Clause 23. The system of any one of clauses 19-22, each coil being disposed within the distal tip and positioned so as to overlap with a respective electrode of the plurality of electrodes along the longitudinal axis.
Clause 24. The system of clause 23, the elongated shaft comprising an outer wall and a lumen wall that defines the lumen, each coil being disposed between the outer wall and the lumen wall.
Clause 25. The system of any one of clauses 19-24, the elongated shaft having an outer diameter between approximately .04 inches and .05 inches.
Clause 26. The system of any one of clauses 19-25, each coil comprising a single axis sensor.
Clause 27. The system of any one of clauses 19-26, the guidewire having a diameter between approximately .01 and .02 inches.
Clause 28. A system comprising: an elongated probe that extends along a longitudinal axis and is dimensioned to be inserted into a coronary sinus, the elongated catheter shaft comprising an opening; a guidewire catheter that extends through the opening along the longitudinal axis and is dimension to be inserted into a vein of Marshall; a first plurality of electrodes connected to the elongated probe along the longitudinal axis; and a second plurality of electrodes connected to the guidewire catheter along the longitudinal axis, each electrode of the first plurality of electrodes and the second plurality of electrodes being configured to (i) generate a current that is indicative of a position of the respective electrode and (ii) sense anatomical signals of a tissue and provide electrical signals which are indicative of the anatomical signals.
Clause 29. The system of clause 28, each electrode further being configured to convey ablation energy to a target tissue region proximal the vein of Marshall.
Clause 30. The system of any one of clauses 28-29, the opening being at a distal end of the elongated probe.
Clause 31. The system of any one of clauses 28-29, the opening being spaced from a distal end of the elongated probe.
Clause 32. The system of any one of clauses 28-31, further comprising an inflatable probe balloon connected to the elongated probe.
Clause 33. The system of any one of clauses 28-32, further comprising an inflatable guidewire balloon connected to the guidewire catheter proximal the second plurality of electrodes.
Clause 34. The system of any one of clauses 28-33, the guidewire catheter having an outer diameter of approximately .03 to .04 inches.
Clause 35. The system of any one of clauses 28-34, the elongated probe having an outer diameter of approximately .08 to .09 inches.
Clause 36. The system of any one of clauses 28-35, further comprising a guidewire, and the guidewire catheter defining a guidewire lumen therethrough, the guidewire extending through the guidewire lumen.
Clause 37. The system of any one of clauses 28-36, the elongated probe and the guidewire being coaxial with the longitudinal axis
Clause 38. A method for navigating a medical probe to a vein of Marshall, the method comprising: inserting an elongated probe that extends along a longitudinal axis into a coronary sinus, the elongated probe defining an opening and comprising a first plurality of electrodes; sliding a guidewire catheter through the elongated probe and out of the opening of the elongated probe into the vein of Marshall, the guidewire catheter comprising a second plurality of electrodes; generating a respective current in each electrode of the first plurality of electrodes and the second plurality of electrodes, the respective current being indicative of a position of each respective electrode; sensing anatomical signals in the vein of Marshall with at least one electrode of the second plurality of electrodes; and providing an electrical signal from the at least one electrode of the second plurality of electrodes which is indicative of the anatomical signals.
Clause 39. The method of clause 38, the anatomical signals comprising electrocardiogram signals.
Clause 40. The method of any one of clauses 38-39, further comprising: conveying ablation energy to a target tissue region proximal the vein of Marshall.
Clause 41. The method of clause 40, the ablation energy comprising unipolar ablation energy that is conveyed by routing direct electrical current between at least one electrode of the second plurality of electrodes and an electrode patch.
Clause 42. The method of clause 40, the ablation energy comprising bipolar ablation energy that is conveyed by routing direct electrical current in a manner selected from the group consisting of: between at least one electrode of the second plurality of electrodes and another electrode of the second plurality of electrodes or the first plurality of electrodes, between at least one electrode of the second plurality of electrodes and a catheter positioned within an atrium, and combinations thereof.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. A medical probe comprising:
an elongated shaft that extends along a longitudinal axis and is dimensioned to be inserted into a vein of Marshall, the elongated shaft comprising a distal tip and defining a lumen;
a guidewire that extends through the lumen;
a plurality of coils connected to the distal dip along the longitudinal axis, each coil being configured to generate a current when subjected to a magnetic field, the current being indicative of a position of the respective coil; and
a plurality of electrodes connected to the distal tip along the longitudinal axis, each electrode being configured to (i) sense anatomical signals in the vein of Marshall and provide electrical signals which are indicative of the anatomical signals or (ii) convey ablation energy to a target tissue region proximal the vein of Marshall.

2. The medical probe of claim 1, each electrode comprising a ring electrode comprising an electrode axis coaxial with the longitudinal axis.

3. The medical probe of claim 1, a first coil of the plurality of coils being disposed within the distal tip and positioned so as to overlap with a distalmost electrode of the plurality of electrodes along the longitudinal axis, optionally the plurality of coils further comprising a second coil and a third coil, the second coil and the third coil being positioned away from the distal end of the distal tip such that the second coil and the third coil are non-overlapping with each electrode of the plurality of electrodes.

4. The medical probe of claim 1, the elongated shaft comprising:
an outer wall; and
a lumen wall that defines the lumen; and
optionally each coil being disposed between the outer wall and the lumen wall.

5. The medical probe of claim 1, the elongated shaft having an outer diameter between approximately .04 inches and .05 inches.

6. The medical probe of claim 1, each coil comprising a single axis sensor selected from a group consisting of a flat spiral coil, cylindrical coil or combination thereof.

7. The medical probe of claim 1, each electrode being configured to (i) sense the anatomical signals in the vein of Marshall and provide the electrical signals which are indicative of the anatomical signals and (ii) convey the ablation energy to the target tissue region proximal the vein of Marshall.

8. A system comprising:
a medical probe comprising:
an elongated shaft that extends along a longitudinal axis and is dimensioned to be inserted into a vein of Marshall, the elongated shaft comprising a distal tip and defining a lumen therethrough;
a guidewire that extends through the lumen;
a plurality of coils connected to the distal dip along the longitudinal axis, each coil being configured to generate a current when subjected to a magnetic field, the current being indicative of a position of the respective coil; and
a plurality of electrodes connected to the distal tip along the longitudinal axis, each electrode being configured to (i) sense anatomical signals in the vein of Marshall and provide electrical signals which are indicative of the anatomical signals or (ii) convey ablation energy to a target tissue region proximal the vein of Marshall; and
a processor configured to estimate a position of the distal tip based on the generated currents and (i) estimate at least one characteristic of the anatomical signals based on the electrical signals or (ii) provide a signal to convey the ablation energy to the electrodes.

9. The system of claim 8, each electrode comprising a ring electrode comprising an electrode axis coaxial with the longitudinal axis.

10. The system of claim 8, each coil being disposed within the distal tip and positioned so as to overlap with a respective electrode of the plurality of electrodes along the longitudinal axis, optionally the elongated shaft comprising an outer wall and a lumen wall that defines the lumen, each coil being disposed between the outer wall and the lumen wall.

11. The system of claim 8, each coil comprising a single axis sensor.

12. A system comprising:
an elongated probe that extends along a longitudinal axis and is dimensioned to be inserted into a coronary sinus, the elongated catheter shaft comprising an opening;
a guidewire catheter that extends through the opening along the longitudinal axis and is dimension to be inserted into a vein of Marshall;
a first plurality of electrodes connected to the elongated probe along the longitudinal axis; and
a second plurality of electrodes connected to the guidewire catheter along the longitudinal axis,
each electrode of the first plurality of electrodes and the second plurality of electrodes being configured to (i) generate a current that is indicative of a position of the respective electrode and (ii) sense anatomical signals of a tissue and provide electrical signals which are indicative of the anatomical signals.

13. The system of claim 12, each electrode further being configured to convey ablation energy to a target tissue region proximal the vein of Marshall.

14. The system of claim 12, further comprising an inflatable probe balloon connected to the elongated probe, or an inflatable guidewire balloon connected to the guidewire catheter proximal the second plurality of electrodes.

15. The system of claim 12, i) the guidewire catheter having an outer diameter of approximately .03 to .04 inches, or ii) further comprising a guidewire, and the guidewire catheter defining a guidewire lumen therethrough, the guidewire extending through the guidewire lumen.
